# EUROPEAN PATENT APPLICATION

(11) **EP 3 305 913 A1**
(43) Date of publication of application: **11.04.2018**
(21) Application number: 16803258.9
(22) Date of filing: 27.05.2016
(51) Int. Cl.: C12Q 1/68, C12N 15/09, C12Q 1/25, G01N 33/50, G01N 33/53

(54) **NUCLEIC ACID DETECTION METHOD, NUCLEIC ACID QUANTITATIVE DETERMINATION METHOD, NUCLEIC ACID BASE SEQUENCE IDENTIFICATION METHOD, NUCLEIC ACID MUTATION OR POLYMORPHISM IDENTIFICATION METHOD, NUCLEIC ACID DETECTION KIT, AND REACTION CHIP**

(30) Priority: 29.05.2015 JP 2015110895
(71) Applicant: Toppan Printing Co., Ltd., Tokyo 110-0016 (JP); Tokyo Institute of Technology, Tokyo 152-8550 (JP)
(72) Inventor: KITANO, Shiro, Tokyo 110-0016 (JP); MARUYAMA, Atsushi, Tokyo 152-8550 (JP); SHIMADA, Naohiko, Tokyo 152-8550 (JP)
(74) Representative: TBK
(86) International application number: PCT/JP2016/065751
(87) International publication number: WO 2016/194823

(57) **Abstract**

The present invention relates to a method for detecting a target nucleic acid using a catalytic nucleic acid, which can improve the reaction efficiency of a catalytic nucleic acid and achieve continuous reaction under isothermal conditions. The method for detecting a target nucleic acid includes a reaction step to obtain a first nucleic acid product by hybridizing a first nucleic acid which is a substrate to a first binding site of a catalytic nucleic acid equipped with an active site having a catalytic action; and a detection step for detecting at least one of the first nucleic acid product or unreacted first nucleic acids, wherein, the hybridization is carried out in the presence of a cationic comb-type polymer.

## Description

### TECHNICAL FIELD

The present invention relates to a method of detecting a nucleic acid by a catalytic action of a catalytic nucleic acid, a method of quantifying a nucleic acid, a method of identifying a base sequence, a method of identifying a mutation or polymorphism of a nucleic acid, a kit for detecting a nucleic acid, and a reaction chip.

Priority is claimed on Japanese Patent Application No. 2015-110895, filed on May 29, 2015, the contents of which are incorporated herein by reference.

### BACKGROUND ART

Over the past two decades, a wide variety of nucleic acid molecules having catalytic activity have been discovered. Single-stranded nucleic acids such as single-stranded DNA and single-stranded RNA form complex three-dimensional structures that can function as catalysts. Although RNA enzymes (ribozymes, RNAzymes) exist in nature, it is also possible to artificially manipulate those so as to specifically recognize the target RNA substrate (Non-Patent Document 1). In addition, the development of many catalytic nucleic acids such as deoxyribonucleic acids often referred to as DNA enzymes (deoxyribozyme, DNAzyme) was encouraged (Non-Patent Document 2).

The types of catalytic reaction of DNAzyme or RNAzyme such as: cleavage of nucleic acids (Non-Patent Documents 3 to 6); ligation of nucleic acids (Non-Patent Documents 7 and 8); ester bonds (Non-Patent Document 9); porphyrin metalation (Non-Patent Document 10); carbon-carbon bonds (Non-Patent Document 11); formation of amide bonds (Non-Patent Document 12); and the like in which ability to catalyze a wide range of reactions has been reported.

In recent years, Elisa Mokany and colleagues developed a method for detecting a target nucleic acid using a catalytic nucleic acid. They enabled to manipulate the catalytic activity of multicomponent nucleic acid complex, and Multicomponent Nucleic Acid Enzyme (MNAzyme) (Patent Document 1).

On the other hand, a method for detecting a nucleic acid using a cationic polymer has been devised (Patent Document 2). This is the method utilizes that if the sequence between the sample nucleic acid and the reference nucleic acid is completely the same, the strand displacement reaction between the single-stranded nucleic acid of the sample and the double-stranded nucleic acid of the reference is fast, and when even the difference is one base, strand displacement reaction is remarkably slow as compared with the case where the sequences are completely the same. Here, the cationic polymer is a polymer obtained by graft polymerization of a cationic polymer such as polylysine, polyarginine or the like as a main chain, and dextran, polyethylene glycol or the like as a side chain. Examples of cationic polymers such as α-PLL-g-Dex, ε-PLL-g-Dex and PAA-g-Dex have been disclosed. It has been reported that the strand exchange reaction of nucleic acids promotes in the presence of the cationic polymer.

In addition, as shown in Patent Document 3, a cationic comb-type copolymer is used as a substance that promotes a chain exchange activity of a single-stranded nucleic acid with respect to a double-stranded short-chain nucleic acid.

### CITATION LIST

### PATENT LITERATURE

[Patent Document 1] Published Japanese Translation No. 2010-505394
[Patent Document 2] Japanese Patent No. 4178194
[Patent Document 3] Japanese Unexamined Patent Publication No. 2008-278779

### NON-PATENT LITERATURE

[Non-Patent Document 1] Haseloff J and Gerlach WL, Nature, August 1988, Vol. 334, No. 6183, p. 585-591
[Non-Patent Document 2] Emilsson GM, Breaker RR. Cellular and Molecular Life Sciences, April 2002, 59, No. 4, p. 596-607
[Non-Patent Document 3] Carmi N, Shultz LA, Breaker RR. Chemistry and Biology, December 1996, Vol. 3, No. 12, p. 1039-1046
[Non-Patent Document 4] Raillard SA, Joyce GF. Biochemistry, September 1996, Volume 35, No. 36, p. 11693-11701
[Non-Patent Document 5] Breaker RR. Current Opinion in Chemical Biology, June 1997, Volume 1, No. 1, p. 26-31
[Non-Patent Document 6] Santoro SW, Joyce GF. Biochemistry, September 1998, Vol. 37, No. 38, p. 13330-13342
[Non-Patent Document 7] Cuenoud B, Szostak JW. Nature, June 1995, Vol. 375, No. 6532, p. 611 - 614
[Non-Patent Document 8] Prior TK, Semlow DR, Flynn-Charlebois A, Rashid I, Silverman SK. Nucleic Acids Research, February 2004, Vol. 32, No. 3, p. 1075-1082
[Non-Patent Document 9] Illangasekare M, Sanchez G, Nickles T, Yarus M. Science, February 1995, Vol. 267, No. 5198 p. 643-647
[Non-Patent Document 10] Li Y, Sen D. Nature Structural Biology, September 1996, Vol. 3, No. 9, p. 743-747
[Non-Patent Document 11] Tarasow TM, Tarasow SL, Eaton BE. Nature, September 1997, Vol. 389, No. 6646, p. 54-57
[Non-Patent Document 12] Lohse PA, Szostak JW. Nature, May 1996, Vol. 381, No. 6581, p. 442-444

### SUMMARY OF THE INVENTION

### PROBLEMS TO BE SOLVED BY THE INVENTION

Although a method for detecting a target nucleic acid using a catalytic nucleic acid such as Patent Document 1 is an excellent method, a temperature difference has to be applied at the time of hybridization and dissociation of the base pair bonding in order to appropriately proceed the reaction. Along with that, a temperature control device for strict temperature control is also required. Further, there is a problem because a large amount of nucleic acid as a substrate is necessary in the reaction of only ordinary catalytic nucleic acid, and it is difficult to detect a trace amount of sample or a trace amount of nucleic acid contained in the sample.

Nucleic acid amplification may be carried out in order to increase the amount of nucleic acid to serve as a substrate, however, it also tends to lead to false positives because a bias in nucleic acid amplification may be caused. Also, since the method requires nucleic acid amplification, expensive materials such as extension enzymes are also required, which leads to an increase in product cost.

Under such circumstances, the present invention provides a method for detecting a nucleic acid for enabling to improve the reaction efficiency of a catalytic nucleic acid in a reaction of a catalytic nucleic acid and a target nucleic acid, a method for quantitatively determining a nucleic acid, a method for identifying a base sequence of a nucleic acid, a method for identifying a mutation or polymorphism of a nucleic acid, a nucleic acid detection kit, and a reaction chip.

### MEANS FOR SOLVING THE PROBLEMS

As a result of intensive research to solve the above problems, the present inventors have found that, the above problems are solved by adding a cationic comb-type polymer to the reaction system in a method for detecting a nucleic acid using a catalytic nucleic acid, and completed the present invention.

That is, the present invention provides the following a method of detecting nucleic acid, a method of quantifying nucleic acid, a method of identifying a nucleic acid base sequence, a method of identifying a mutation or polymorphism of a nucleic acid, a kit for detecting a nucleic acid, and a reaction chip.
[1] A method for detecting a nucleic acid including a reaction step to obtain a first nucleic acid product by hybridizing a first nucleic acid which is a substrate to a first binding site of a catalytic nucleic acid equipped with an active site having a catalytic action; and a detection step for detecting at least one of the first nucleic acid product or a unreacted first nucleic acid, wherein, the hybridization is carried out in the presence of a cationic comb-type polymer.
[2] The method for detecting a nucleic acid according to [1], further including a step for dissociating hybridization between the first nucleic acid product hybridized to the first binding site of the catalytic nucleic acid in the reaction step.
[3] The method for detecting a nucleic acid according to [1] or [2], wherein the catalytic nucleic acid further equips a second binding site capable of hybridizing to a second nucleic acid, hybridizing the second nucleic acid to the second binding site causes the catalytic action of the catalytic nucleic acid in the reaction step, and detecting the second nucleic acid by detecting at least one of the first nucleic acid product or unreacted first nucleic acids in the detection step.
[4] The method for detecting a nucleic acid according to any one of [1] to [3], wherein the cationic comb-type copolymer having a main chain which is a polymer chain containing a cationic group and a side chain which is a hydrophilic group.
[5] The method for detecting a nucleic acid according to [4], wherein the main chain of the cationic comb-type copolymer is polylysine.
[6] The method for detecting a nucleic acid according to [4] or [5], wherein the side chain of the cationic comb-type copolymer is dextran.
[7] The method for detecting a nucleic acid according to any one of [4] to [6], wherein the molecular weight of the main chain portion of the cationic comb-type copolymer is 5,000 or more.
[8] The method for detecting a nucleic acid according to any one of [1] to [7], wherein the reaction step is carried out in the presence of a divalent metal ion.
[9] The method for detecting a nucleic acid according to [8], wherein the divalent metal ion is magnesium ion or manganese ion.
[10] The method for detecting a nucleic acid according to any one of [2] to [9], a step of hybridizing the first nucleic acid to the first binding site, a step for dissociating hybridization between the first nucleic acid product hybridized to the first binding site of the catalytic nucleic acid and the catalytic nucleic acid, wherein these steps are conducted under conditions of a temperature difference of within 40°C.
[11] The method for detecting a nucleic acid according to any one of [1] to [10], wherein the reaction step is carried out within a temperature range of 20°C to 70°C.
[12] The method for detecting a nucleic acid according to any one of [1] to [11], wherein the first nucleic acid is labeled with a labeling substance.
[13] The method for detecting a nucleic acid according to [12], wherein the first nucleic acid is labeled with a first labeling substance and a second labeling substance which are different from each other, and transferring energy between the first labeling substance and the second labeling substance.
[14] The method for detecting a nucleic acid according to [13], wherein the labeling substance is a fluorescent substance.
[15] The method for detecting a nucleic acid according to [13] or [14], wherein the first labeling substance and the second labeling substance are a combination of a fluorescent substance and a quenching substance.
[16] The method for detecting a nucleic acid according to any one of [1] to [15], wherein the first nucleic acid or the second nucleic acid to be detected is a nucleic acid at least one selected from a group consisting of DNA, methylated DNA, alkylated DNA, RNA, microRNA, siRNA, mRNA, tRNA, snoRNA, noncoding RNA, the derivative thereof, the amplicon thereof, and the complexes thereof.
[17] The method for detecting a nucleic acid according to any one of [1] to [16], wherein the first nucleic acid or the second nucleic acid to be detected is derived from peripheral blood, serum, plasma, saliva, buccal swab, FFPE (paraffin embedded) section or tissue.
[18] A method for quantifying a nucleic acid characterized by measuring the amount of at least one of the first nucleic acid product or unreacted first nucleic acids detected by the nucleic acid detection method according to any one of [1] to [17].
[19] A method for identifying a base sequence of a nucleic acid, including a reaction step of obtaining a first nucleic acid product by hybridizing a first nucleic acid which is a substrate to a first binding site of a catalytic nucleic acid equipped with a catalytic active site having a catalytic action; a detection step of detecting at least one of the first nucleic acid product or unreacted first nucleic acids; and an identification step of identifying the base sequence of the nucleic acid based on the measurement result obtained in the detection step, wherein the hybridization is carried out in the presence of a cationic comb-type polymer.
[20] The method for identifying a base sequence of a nucleic acid according to [19], the catalytic nucleic acid further equips a second binding site capable of hybridizing with the second nucleic acid, hybridizing the second nucleic acid to the second binding site causes the catalytic action of the catalytic nucleic acid in the reaction step, and detecting the second nucleic acid by detecting at least one of the first nucleic acid product or unreacted first nucleic acids in the detection step.
[21] A method for identifying a mutation or polymorphism of a nucleic acid, characterized by identifying a mutation or polymorphism in a base sequence of a nucleic acid to be detected by the method for identifying a base sequence of a nucleic acid according to [19] or [20].
[22] The method for identifying a mutation or polymorphism of a nucleic acid according to [21], wherein the mutation or polymorphism of a base sequence of a nucleic acid to be detected by comparing the amount of the first nucleic acid product and unreacted first nucleic acids.
[23] A kit for detecting a nucleic acid including the cationic comb-type polymer, wherein the kit is used for the method for detecting a nucleic acid according to any one of [1] to [17].
[24] A reaction chip for using the method for detecting a nucleic acid according to any one of [1] to [17], wherein the reaction chip preliminary holds at least one of the
   cationic comb-type copolymer or the catalytic nucleic acid, and the reaction chip includes a reaction well for holding the cationic comb-type copolymer and the catalytic nucleic acid.

### EFFECT OF THE INVENTION

According to the method for detecting a nucleic acid of the present invention, in the method for detecting a target nucleic acid using a catalytic nucleic acid, it is capable of improving the reaction efficiency of the catalytic nucleic acid and even a trace amount of nucleic acid which could not be detected by the conventional method can be detected easily in a short time.

### BRIEF DESCRIPTION OF THE DRAWINGS

FIG. 1 is a diagram schematically showing an example of a state of reaction between a catalytic nucleic acid and a nucleic acid substrate used in a nucleic acid detection method of the present invention.
FIG. 2 is a diagram schematically showing an example of a reaction state between a catalytic nucleic acid and a nucleic acid substrate used in the nucleic acid detection method of the present invention.
FIG. 3A is a diagram comparing the state of temperature control between the method for detecting a nucleic acid of the present invention and that of the conventional method.
FIG. 3B is a diagram comparing the state of temperature control between the method of detecting a nucleic acid of the present invention and that of the conventional method.
FIG. 4 is a plan view showing one embodiment of a reaction chip according to the present invention.
FIG. 5 is a diagram showing sequences of DNAzymes and sequences of target nucleic acids used in Examples 1 and 2.
FIG. 6 is a diagram showing the sequence of the MNAzyme, the sequence of the target nucleic acid, and the sequence of the nucleic acid substrate used in Examples 3 to 4.
FIG. 7 is an electrophoretic image showing nucleic acid substrate and nucleic acid substrate products of each reaction solution after a lapse of a predetermined time from the start of the reaction in Example 1-1.
FIG. 8 is an electrophoretic image showing nucleic acid substrate and nucleic acid substrate products of each reaction solution after a lapse of a predetermined time from the start of the reaction in Example 1-2.
FIG. 9 is a graph comparing an adaptation temperature range depending on the presence or absence of a cationic comb-type polymer in a reaction solution in Example 2.
FIG. 10 is a graph indicating how the fluorescence intensity of a reaction solution changes before and after the addition of a cationic comb-type polymer in Example 3.
FIG. 11 is a graph indicating the difference in cleavage efficiency of MNAzyme substrate with or without cationic comb-type copolymer at each target nucleic acid concentration in Example 4-1.
FIG. 12 is a graph indicating the difference in cleavage efficiency of MNAzyme substrate with or without cationic comb-type copolymer at each target nucleic acid concentration in Example 4-2.

### DETAILED DESCRIPTION OF THE INVENTION

### « Method of Detecting Nucleic Acids »

A method for detecting a nucleic acid of the present invention including a reaction step to obtain a first nucleic acid product by hybridizing a first nucleic acid which is a substrate to a first binding site of a catalytic nucleic acid equipped with an active site having a catalytic action; and a detection step for detecting the first nucleic acid product and/or unreacted first nucleic acids, wherein, the hybridization is carried out in the presence of a cationic comb-type polymer.

As an example of "the hybridization is carried out in the presence of a cationic comb-type polymer", it may be "the hybridization is carried out in a solution to which a cationic comb-type polymer is added".

The nucleic acid detection method in the embodiment described below is a method of detecting whether or not the nucleic acid to be detected is included in the sample, by detecting whether or not a first nucleic acid which can be a substrate of a catalytic nucleic acid has reacted by catalytic action of a catalytic nucleic acid to detect. The nucleic acid to be detected may be the first nucleic acid as a substrate itself or another nucleic acid to be detected may be detected through detection of the first nucleic acid. As another nucleic acid, a second nucleic acid to be described later can be included.

### (First Embodiment)

### <Reaction Process>

The method of detecting a nucleic acid of the present invention includes a reaction step to obtain a first nucleic acid product by hybridizing a first nucleic acid which is a substrate to a first binding site of DNAzyme equipped with an active site having a catalytic action.

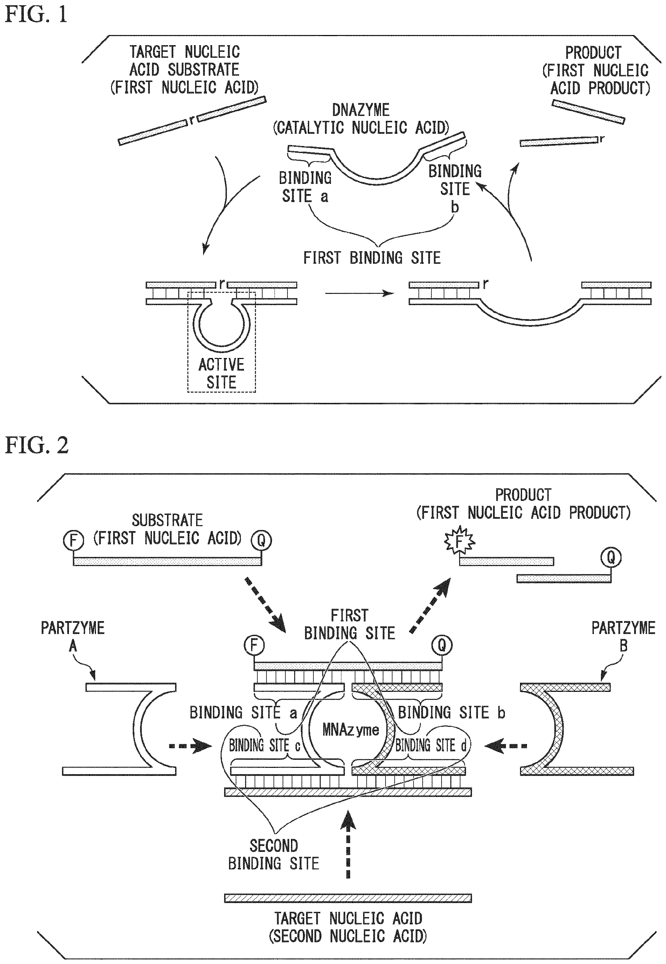

FIG. 1 is a diagram schematically showing an example of a reaction between a catalytic nucleic acid and a nucleic acid substrate in the first embodiment. The DNAzyme (catalytic nucleic acid) has a first binding site consisting of a sequence complementary to the first nucleic acid as a substrate. The first binding site is hybridizable to the first nucleic acid. The DNAzyme has an active site having a catalytic activity, and the active site has an activity capable of cleaving the first nucleic acid. The first binding site is divided into two regions such as a binding site a and a binding site b with the active site therebetween.

The DNAzyme can be artificially synthesized by a known method such as a solid phase synthesis method according to the sequence of the substrate and the target nucleic acid.

In order to increase the reaction rate, the substrate binding site is preferably arranged within 3 bases to 15 bases from the 3' end and/or the 5' end.

In addition, since the target binding site corresponds to various target nucleic acids, it is preferable that the target binding site is arranged at 4 bases to 20 bases from the 3' end and/or the 5' end.

The length of the DNAzyme is not particularly limited, and it can be adjusted to a desired base length.

In the reaction step, it is preferable to include both a step of hybridizing the first nucleic acid to the first binding site of the catalytic nucleic acid (hereinafter, referred to as a binding step) and a step of dissociating hybridization between the catalytic nucleic acid and the first nucleic acid product hybridized to the first binding site of the catalytic nucleic acid (hereinafter, referred to as a dissociation step). In the reaction step, the binding step and the dissociation step may be repeated.

In the present embodiment, it is preferable that the reaction step includes both a step of hybridizing the DNAzyme and the first nucleic acid, and a step of dissociating hybridization between the DNAzyme and the first nucleic acid product.

By hybridization of the first nucleic acid to the first binding site such as the binding site a and the binding site b, the first nucleic acid is cleaved by the catalytic action of the active site, resulting in the product (first nucleic acid product). When the first nucleic acid product dissociates from the first binding site, the first binding site of the DNAzyme can hybridize with unreacted first nucleic acids, which is a new substrate. In this way, the DNAzyme functions as a multi-turnover enzyme that continuously reacts.

The method of detecting a nucleic acid of the present embodiment which includes a step of hybridizing a DNAzyme with a first nucleic acid and a step of dissociating hybridization between the DNAzyme and the first nucleic acid product, is conducted in the presence of a cationic comb-type polymer.

Note that as an example of "a step of hybridizing a DNAzyme with a first nucleic acid and a step of dissociating hybridization between the DNAzyme and the first nucleic acid product, is conducted in the presence of a cationic comb-type polymer", it may be "a step of hybridizing a DNAzyme with a first nucleic acid and a step of dissociating hybridization between the DNAzyme and the first nucleic acid product, is conducted in a solution to which the cationic comb-type polymer is added".

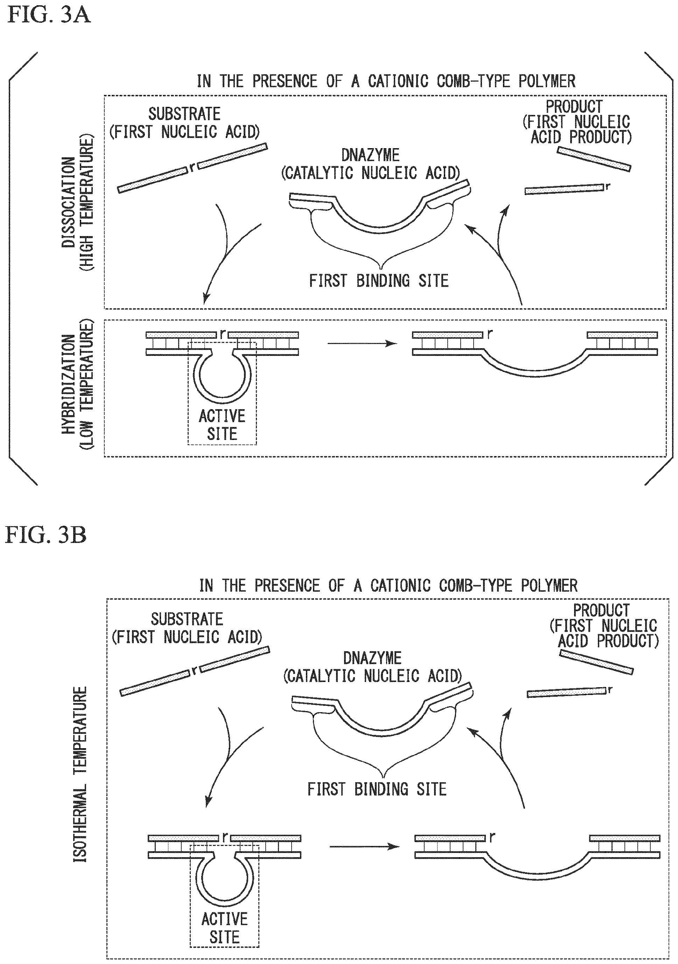

FIG. 3A and 3B is a diagram schematically showing an example of a reaction between a catalytic nucleic acid and a nucleic acid substrate in the method for detecting a nucleic acid of the first embodiment and that of the conventional method. FIG. 3A shows a conventional method, and FIG. 3B shows a nucleic acid detection method of the first embodiment.

As shown in FIG. 3A, hybridization of the catalytic nucleic acid and the nucleic acid substrate was carried out at a low temperature in the reaction between the conventional catalytic nucleic acid and the nucleic acid substrate. On the other hand, dissociation of base pair binding between catalytic nucleic acid and nucleic acid substrate was carried out at a higher temperature. This promotes substrate turnover.

On the other hand, a cationic comb-type polymer is added to the reaction system in the nucleic acid detection method of the first embodiment shown in FIG. 3B. If hybridization and dissociating hybridization are carried out in the presence of a cationic comb-type polymer, hybridization of the catalytic nucleic acid and the nucleic acid substrate and dissociation of base pair binding between catalytic nucleic acid and nucleic acid substrate would be promoted. Therefore, since the turnover of the substrate is promoted, the reaction rate is remarkably increased, and the reaction can be carried out efficiently in a short time.

### <Detecting Step>

The method of detecting nucleic acid of the present embodiment includes a detection step of detecting the first nucleic acid product and/or unreacted first nucleic acids.

As described in the reaction step, if the first nucleic acid having a target base sequence hybridizable with the first binding site is contained in the reaction system, the first nucleic acid is cleaved by the catalytic action of the catalytic nucleic acid, and the first nucleic acid product is produced. On the other hand, if the first nucleic acid is not contained in the reaction system, the first nucleic acid product is not produced. In this manner, it is possible to detect whether or not the first nucleic acid to be detected is present in the reaction system by detecting a value indicating the presence of the first nucleic acid product. Further, by detecting a value indicating the presence of the first nucleic acid product and/or unreacted first nucleic acids, it is possible to detect whether or not the first nucleic acid to be detected is present in the reaction system. From the presence of the first nucleic acid product, it is also possible to detect that the first nucleic acid is a nucleic acid capable of reacting with the catalytic nucleic acid.

The detection step may be carried out at the end of the reaction step or at the start and end of the reaction step. For example, the first nucleic acid product and/or unreacted first nucleic acids may be measured by comparing measured values before and after the reaction step. Further, in the reaction step, the detection step may be carried out over time (in real time). When the reaction step and the detection step are carried out in real time, fluorescence change or the like may be sequentially measured using a known real time-PCR apparatus or the like.

Reacting the first nucleic acid by the catalytic action of the catalytic nucleic acid means, for example, cleaving the first nucleic acid by the catalytic action of the catalytic nucleic acid, ligating the first nucleic acid with another nucleic acid, ester binding of the first nucleic acid and another molecule, carbon-carbon bonding the first nucleic acid and another molecule, amide bonding the first nucleic acid and another molecule, and the like.

In the detection step, the first nucleic acid product and unreacted first nucleic acids are detected in separate, or only either one of the first nucleic acid product and unreacted first nucleic acids is detected or preferentially detected. The method is not particularly limited.

For example, in the reaction catalyzed by the catalytic nucleic acid, the first nucleic acid product and unreacted first nucleic acids can be distinguished by the length of the nucleic acid, as an example. Identification of the length of the nucleic acid can be performed by a known method such as electrophoresis.

In the case of the conventional method for detecting nucleic acid,, the rate of turnover is slow in the reaction at a constant temperature.

In addition, usually about 70 nM of the target nucleic acid is required, and the number of copy in 70 nM of usual sample amount is about 4 × 10¹⁰ copies.

In order to practically obtain the number of copy corresponding to this copy number, amplification of nucleic acid by PCR or isothermal amplification or the like becomes indispensable. This point is a bottleneck in practical use of a catalytic nucleic acid because it causes the increment of a measurement time or cost due to a nucleic acid amplification step. Further, a device for controlling strict temperature control is also required in the nucleic acid amplification step and the turnover step of the catalytic nucleic acid and the substrate.

On the other hand, according to the nucleic acid detection method of the first embodiment, the reaction can be carried out under isothermal conditions because hybridization of the catalytic nucleic acid with the nucleic acid substrate and dissociation of the base pair bond between the catalytic nucleic acid and the nucleic acid substrate are repeated without repeating temperature changes of high temperature and low temperature of a reaction system (for example, reaction solution). Furthermore, the temperature range of the catalytic activity adaptation of catalytic nucleic acid can be expanded by adding a cationic comb-type copolymer.

In the detection reaction of a target nucleic acid using a catalytic nucleic acid, temperature control is conventionally required. This is a barrier to the practical application of a detection reaction of a target nucleic acid using a catalytic nucleic acid. However, according to the method of the present invention, strict temperature control is not necessary in the detection reaction of a target nucleic acid using a catalytic nucleic acid, and the present invention enables to provide an innovative method to eliminate barriers for practical use up to now.

In addition, it is possible to detect a sample without carrying out a nucleic acid amplification step even on a sample containing a low concentration of a target nucleic acid, which conventionally cannot be detected unless the nucleic acid amplification step is carried out.

The methods for amplifying target nucleic acids have been widely used in the research/clinical field. However, regardless of their capabilities, each method has its own disadvantages. That is, it requires protein enzymes such as DNA polymerase, RNA polymerase and reverse transcriptase and the like. The inclusion of protein enzymes requires consideration of the complexity of the protocol, the influence of inhibitors and the influence of pH. In addition, there are problems that cost increases due to expensive protein enzymes, storage stability of reagents and kits, and product life are inevitably shortened.

According to the nucleic acid detection method of the first embodiment, since the method uses catalytic nucleic acid, these problems associated with protein enzymes can be avoided.

Other related technical problems are likely to lead to false positives due to false positive detection due to contamination of reaction systems of other amplified products and amplification of wrong base insertion due to typical error of the enzyme. Such a target nucleic acid amplification reaction tends to cause such problem because it exponentially dramatically amplifies the template. Furthermore, depending on the detection system of the assay, there is a possibility that the dimerization of the primer may become a false positive. Therefore, the target nucleic acid amplification reaction using the enzyme has various bias factors, which may make it difficult to detect the target nucleic acid.

On the other hand, in the nucleic acid detection method of the first embodiment, as described in the following examples, the detection sensitivity of nucleic acid was improved by about 35,000 times compared with the conventional method by conducting the hybridization in the presence of a cationic comb-type polymer. This improvement in detection sensitivity is astonishing, and it is possible to directly and accurately detect without a nucleic acid amplification step even a low concentration nucleic acid which was impossible to detect directly by conventional methods.

As described above, the nucleic acid detection method of the present embodiment dramatically improves the reactivity as compared with the conventional method while maintaining the degree of accuracy that can detect the difference in the base sequence of the target nucleic acid due to the hybridizing property of the nucleic acid. In addition, the nucleic acid detection method of the present embodiment is capable of detecting whether or not the nucleic acid contained in the sample is a nucleic acid having a target base sequence in a very short time without requiring a nucleic acid amplification process. These reactivity enhancement levels are revolutionary to overcome the common sense of nucleic acid detection methods using conventional catalytic nucleic acids.

### (Second Embodiment)

### <Reaction Step>

The nucleic acid detection method of the second embodiment includes a reaction such that a DNAzyme has a first binding site capable of hybridizing with a first nucleic acid as a substrate and an active site having a second binding site catalytic action capable of hybridizing with a second nucleic acid, then the second nucleic acid is hybridized to the second binding site of the DNAzyme causes catalysis of DNAzyme to be active. Then, hybridize the first nucleic acid to the first binding site and reacting the first nucleic acid by the catalytic action of the active site to obtain a first nucleic acid product. In the detecting step to be described later, the second nucleic acid is detected by detecting the first nucleic acid product and/or unreacted first nucleic acids.

In the nucleic acid detection method of the second embodiment, description of the points described in the first embodiment is omitted.

FIG. 2 is a diagram schematically showing an example of the reaction between the catalytic nucleic acid and the nucleic acid substrate in the second embodiment. The catalytic nucleic acid MNAzyme (multicomponent nucleic acid enzyme) of the present invention is composed of two partzymes A and B. Each partzyme A, B has a part of a first binding site which is a site capable of hybridizing with the first nucleic acid, and the first binding site is composed of a binding site a and a binding site b.

In addition, each partzyme A and partzyme B has a part of a second binding site which is a site capable of hybridizing with the second nucleic acid, and the second binding site comprises a binding site c and a binding site d.

In the second embodiment, the nucleic acid to be detected for the presence or absence of mutation or the like is the second nucleic acid (target nucleic acid). The nucleic acid sequences of the second binding sites c and d of the partzymes A and B can be determined so that the second nucleic acid to be detected can be hybridized.

Depending on the substrate or the sequence of the target nucleic acid, the partzyme can be artificially synthesized using a known method such as a solid phase synthesis method.

In order to increase the reaction rate, the substrate binding site is preferably arranged within 3 bases to 15 bases from the 3 'end and/or the 5' end.

In addition, it is preferable that the target binding site is arranged at 4 bases to 20 bases from the 3 'end and/or the 5' end since the target binding site corresponds to various target nucleic acids.

The length of the partzyme is not particularly limited, and it can be adjusted to a desired base length.

When the second nucleic acid hybridizes to the binding site c of the partzyme A and the binding site d of the partzyme B and the first nucleic acid hybridizes to the binding site a of the partzyme A and the binding site b of the partzyme B, partzyme A, B assemble to form MNA complexes, which leads to the organization of active MNAzymes that cleaves substrates. Note that hybridization of the second nucleic acid to the second binding site and hybridization of the first nucleic acid to the first binding site may be carried out at the same time; or after the second nucleic acid hybridizes to the second binding site, the first nucleic acid may hybridize to the first binding site; or after the first nucleic acid hybridizes to the first binding site, the second nucleic acid may hybridize to the second binding site.

When the first nucleic acid hybridizes to the first binding site and the second nucleic acid hybridizes to the second binding site, the first nucleic acid is cleaved by the catalytic action of the active site to produce a product (first nucleic acid product). In this manner, cleavage of the first nucleic acid is enabled by hybridization of the second nucleic acid to the second binding site. Therefore, the presence of the second nucleic acid can be detected through detection of the first nucleic acid (first nucleic acid product).

In the reaction step, it is preferable to include both a step of hybridizing the first nucleic acid to the first binding site of the catalytic nucleic acid and hybridizing the second nucleic acid to the second binding site of the catalytic nucleic acid (hereinafter, referred to as a binding step) and a step of dissociating hybridization between the catalytic nucleic acid and the first nucleic acid product hybridized to the first binding site of the catalytic nucleic acid and dissociating hybridization between the catalytic nucleic acid and the second nucleic acid product hybridized to the second binding site of the catalytic nucleic acid (hereinafter, referred to as a dissociation step). In the reaction step, the binding step and the dissociation step may be repeated.

In the reaction step of the present embodiment, it is preferable to include both a step of hybridizing the MNAzyme, the first nucleic acid, and the second nucleic acid (hereinafter sometimes referred to as a binding step) and the dissociating the hybridization between the MNAzyme, the first nucleic acid product and the second nucleic acid (hereinafter sometimes referred to as dissociation step).

When the first nucleic acid product is dissociated from the first binding site, the first binding site of the MNAzyme becomes re-hybridizable with the nucleic acid and enables to hybridize with unreacted first nucleic acids as a new substrate. In addition, then the second nucleic acid product dissociates from the second binding site, the first binding site of the MNAzyme becomes re-hybridizable with the nucleic acid and enables to hybridize with the new second nucleic acid. In this way, another substrate repeats reacting with the MNAzyme (turnover).

### <Detection Step>

In the nucleic acid detection method of the second embodiment, the first nucleic acid is labeled with a labeling substance. For example, in order to investigate whether the first nucleic acid remains intact or cleaved a label capable of detecting whether the two labels are in close proximity or in a separate position may be used.

Fluorescence resonance energy transfer (FRET) is most frequently used for detection in such a case, the first nucleic acid is labeled with a first labeling substance and a second labeling substance which are different from each other, and energy transfer is possible between the first labeling substance and the second labeling substance. Energy transfer between labeling substances means that when at least two types of labeling substances, a donor labeling substance that generates energy and an acceptor labeling substance that absorbs energy generated from the donor labeling substance are in close proximity to each other, refers to the transfer of energy from the donor labeling substance to the acceptor labeling substance. For example, when the two kinds of labeling substances are fluorescent substances, the acceptor labeling substance absorbs the fluorescence generated by exciting the donor labeling substance, the fluorescence emitted by the acceptor labeling substance is measured, or quenching of the donor labeling substance caused by the acceptor labeling substance absorbing the fluorescence generated by exciting the donor labeling substance can be measured (PCR Methods and applications 4,357-362 (1995), Nature Biotechnology 16,49-53 (1998)).

Although energy transfer may occur even if there is no overlap between the fluorescence wavelength of the donor labeling substance and the absorption wavelength of the acceptor labeling substance, such energy transfer is also included in the present invention. For example, in the first nucleic acid as seen from the site to be cleaved of the first nucleic acid, the 3' end is labeled with a fluorescent substance and the 5' end is labeled with another fluorescent substance. When the first nucleic acid is uncleaved, the two fluorescent substances are capable of fluorescence resonance energy transfer, but when the first nucleic acid is cleaved, the two fluorescent substances become incapable of fluorescence resonance energy transfer. Therefore, it is possible to know the signal due to cleavage by measuring the fluorescence resonance energy transfer. Specifically, in the present invention, the 5' end and the 3' end indicate within 30 bases from the 5' end and the 3' end of the nucleic acid strand respectively, but if both of the labeling substances are close to each other, energy transfer is easily caused, therefore, it is preferably within 10 bases from the site to be cleaved of the first nucleic acid.

As a fluorescent substance in this case, it is possible to combine various fluorescent substances, examples of which include fluorescein and tetramethylrhodamine. Also, cleavage of the chain can be detected by labeling one with a fluorescent substance and the other with a quenching substance. In this case, when the fluorescent substance is in the vicinity, it does not emit light, and when the fluorescent substance and the quenching substance separate (when the chain is broken), it emits light. A possible combination of a fluorescent substance and a quenching substance can be various, for example, combination of fluorescein or tetramethylrhodamine as a fluorescent substance, dabsyl or a black hole quencher as a quenching substance, or the like can be combined.

There are no particular restrictions on the two kinds of labeling substances capable of energy transfer with each other as long as they can transfer energy in close proximity to each other. Among them, a fluorescent substance and a delayed fluorescent substance are preferable, and in some cases, a chemiluminescent substance, a bioluminescence substances and the like can also be used. Examples of such a combination of labeling substances include fluorescein and its derivatives (such as fluorescein isothiocyanate and the like) and rhodamine and its derivatives (for example, tetramethylrhodamine isothiocyanate, tetramethylrhodamine-5- (and-6-) hexanoic acid and the like), a combination of fluorescein and DABCYL, and the like, and any combination can be selected from among these (Nonisotopic DNA Probe Techniques. Academic Press (1992)).

In addition, it may be a combination of molecules that release thermal energy when placed in proximity. Examples of such combination of labeling substances include Alexa Fluor (registered trademark) 488 (manufactured by Invitrogen), ATTO 488 (manufactured by ATTO-TEC GmbH), Alexa Fluor (registered trademark) 594 (manufactured by Invitrogen), and ROX Carboxy-X-rhodamine) and a combination of BHQ (registered trademark, Black hole quencher) -1 or BHQ (registered trademark) -2 and the like can be included.

Since guanine is capable of quenching when FAM comes close (Nucleic acids Research 2002, vol. 30, no. 9 2089-2195), it may be used. For example, when the 3' end of one strand of a standard double-stranded nucleic acid is labeled with FAM and the base at the 5' end of the other chain is guanine, the other strand may not be labeled with a labeling substance.

According to the nucleic acid detection method of the second embodiment, in addition to the action and effect of the nucleic acid detection method of the first embodiment, the effect of stabilizing the complex of the catalytic nucleic acid and the substrate is exerted.

In the case of a catalytic nucleic acid which forms an active form by forming a complex such as MNAZyme as in the method of detecting a nucleic acid of the second embodiment, there is a tendency that the reactivity is lowered by not being stabilized the complex, and has been a problem for practical application. However, conducting hybridization in the presence of a cationic comb-type polymer makes it possible to stably form a complex of a catalytic nucleic acid and a substrate, dramatically improving subsequent turnover of the substrate cleavage.

### (Catalytic nucleic acid)

The catalytic nucleic acid used in the nucleic acid detection method of the first embodiment is not particularly limited as long as the catalytic nucleic acid has a first binding site and an active site. The catalytic nucleic acid used in the nucleic acid detection method of the second embodiment is not particularly limited as long as it has a first binding site, a second binding site and an active site. For example, in addition to the catalytic nucleic acids shown in FIGS. 1 and 2, hammerhead type ribozyme, hairpin type ribozyme, "10:23" DNAzyme, "8:17" DNAzyme, "7Z81" ligase and "7Z48" ligase can be included.

"10:23" and "8:17" DNAzymes cleave nucleic acid substrates at specific RNA phosphodiester linkages to produce reaction products with 2', 3'-cyclic phosphate groups and 5'-hydroxyl groups (Refer to Non-Patent Document 5, Non-Patent Document 1). Examples of deoxyribozymes capable of ligating 2', 3'-cyclic phosphate products and 5'-hydroxyl products include "7Z81 ", "7Z48" ligase (see Non-Patent Document 7).

Several catalytic nucleic acids, including hammerhead ribozymes, 10: 23, 8: 17 DNAzyme, "7Z81" and "7Z48" ligase, have similar basic structures with multiple domains. These nucleic acid enzymes have a catalytic domain (catalytic core) sandwiched between two non-conserved substrate binding domains (arms) that specifically recognize a substrate and hybridize therewith. These enzymes can function as multi turnover enzymes.

### (Cationic comb-type polymer)

The cationic comb-type polymer used in the present invention is not particularly limited as long as a side chain is introduced in a comb-type form with the main chain and a polymer containing a cationic group. The cationic comb-type polymer used in the present invention is a polymer obtained by introducing a hydrophilic group in a comb-type form as a side chain to a polymer having a polymer chain (polycation) containing a cationic group as the main chain, and it is more preferable that it is a copolymer in which a polymer containing a hydrophilic group is introduced in a comb-type form into a polycation as a main chain (hereinafter referred to as a cationic comb-type copolymer).

As the polycation which is the main chain of the cationic comb-type copolymer, one type of cationic monomer such as amino acids such as lysine, arginine, histidine, ornithine, sugar such as glucosamine, allylamine, ethyleneimine or the like, polymers obtained by polymerizing plural types, and derivatives thereof. In the present invention, it is preferable to use a cationic comb-type copolymer including polylysine, polyarginine, a copolymer of lysine and arginine, or a derivative thereof

As the derivative of the polymer of the cationic monomer, for example, one obtained by adding a functional group to a part or all of the monomers constituting the polymer and the like can be included. Such a functional group is not particularly limited as long as it does not impair the cationic nature of the polymer, and examples thereof include a guanidyl group and the like.

Examples of the polymer having a hydrophilic group which is a side chain of the cationic comb-type copolymer include polysaccharides such as dextran, amylose and cellulose; polyethers such as polyethylene glycol; and derivatives thereof In the present invention, it is preferable to use a cationic comb-type copolymer having dextran, polyethylene glycol, or a derivative thereof as a side chain.

The derivative of a polymer containing a hydrophilic group used as a side chain is not particularly limited as long as it does not impair the hydrophilicity, and examples thereof include carboxymethyl derivatives, amino acid derivatives, aldehyde derivatives and the like.

The cationic comb-type polymer used in the present invention is preferably a cationic comb-type copolymer in which the main chain is polylysine or polyarginine or a derivative thereof and the side chain is dextran; more preferably a cationic comb-type copolymer in which the main chain is polylysine or polyarginine or a guanidine derivative thereof (derivative containing a guanidyl group) and the side chain is dextran; and most preferably, a cationic comb-type copolymer is obtained by graft polymerizing dextran to polylysine or guanidized polylysine..

The size of the cationic comb-type polymer used in the present invention may be appropriately selected according to the chain length of the sample double-stranded nucleic acid or standard double-stranded nucleic acid used for hybridization. In the present invention, for example, in the case of using a cationic comb-type copolymer obtained by graft polymerizing dextran to polylysine or guanidized polylysine, the molecular weight of the main chain moiety is preferably from 2,000 to 20,000, more preferably from 5,000 to 15,000. A cationic comb-type polymer having a main chain having a desired size can be obtained by adjusting the degree of polymerization of the polycation of the main chain.

### (Reaction Conditions in Reaction Process)

The cationic comb-type polymer may be added to the reaction system (for example, a reaction solution) after starting the reaction step, however, it is preferably added to the reaction system before the start of the reaction step. The amount of the cationic comb-type polymer to be added to the reaction system is determined by the ratio of the cationic comb-type polymer to the nucleic acid ([charge of cationic comb-type polymer] / [charge of nucleic acid]) in the reaction system. An appropriate value within the range of the charge ratio is in the range of 0.01 to 10,000, preferably in the range of 0.1 to 100, more preferably 0.1 to 10, most preferably within the range of 1 to 10. Note, "Charge of nucleic acid" is the total charge of all nucleic acids present in the reaction system.

The amount of the cationic comb-type polymer to be added to the reaction system can also be determined based on the number of phosphate groups of the nucleic acid in the reaction system. Similarly, the amount of cationic comb-type polymer to be added to the reaction system can also be determined based on the number of cationic groups of the cationic polymer, instead of calculating the charge of the cationic comb-type polymer. For example, when the cationic comb-type polymer has an amino group, the amount of the cationic comb-type polymer to be added to the reaction system is determined based on [the amino group of the cationic comb-type polymer] / [the phosphate group of the nucleic acid]). It is preferable that the molar ratio of the group [amino group of cationic comb-type polymer] / [phosphate group of nucleic acid] is preferably in the range of 0.1 to 100, more preferably in the range of 0.1 to 10, and most preferably in the range of 1 to 10. Note that the "phosphate group of a nucleic acid" is the sum of the phosphate groups of all the nucleic acids present in the reaction system.

Besides, in addition to the reaction system, a buffer for adjusting the pH of the reaction system, a monovalent or divalent cation necessary for forming a duplex, an organic solvent or the like which affects the stability of the double-stranded nucleic acid, can be added. As the buffering agent, for example, Tris hydrochloric acid and the like can be used. Examples of the cation include sodium ion, magnesium ion and the like. Examples of the organic solvent include dimethylsulfoxide (DMSO), dimethylformamide (DMF), and the like.

It has been found that the rate of catalytic cleavage of many nucleic acid enzymes depends on the presence and concentration of divalent metal ions such as, for example, Ba²⁺, Sr²⁺, Mg²⁺, Ca²⁺, Mn²⁺, Zn²⁺, Ni²⁺, Co²⁺, Zn²⁺ and the like. Generally, in order to obtain the activity of a catalytic nucleic acid, a divalent metal ion is necessary, however, the interaction between the cationic comb-type copolymer and the DNA does not affect the catalytic activity at all, that is, does not inhibit the interaction between DNA and divalent metal ion.

In the reaction step, it is preferable to include both a step of hybridizing a nucleic acid capable of hybridizing to a catalytic nucleic acid to the catalytic nucleic acid (hereinafter, referred to as a binding step) and a step of dissociating hybridization between the catalytic nucleic acid and the nucleic acid. Both the binding step and the dissociation step are preferably carried out isothermally.

The nucleic acid hybridizing to the catalytic nucleic acid is represented by the first nucleic acid and the second nucleic acid described in the above embodiment.

The temperature range meaning isothermal is defined by the difference between the operating temperature of the binding step and the operating temperature of the dissociation step. The temperature at which the binding step is carried out is the temperature at which the nucleic acid hybridizing with the catalytic nucleic acid is hybridized. The temperature at which the dissociation step is carried out is a temperature at which hybridization between the catalytic nucleic acid and the nucleic acid is dissociated.

A nucleic acid capable of binding to a catalytic nucleic acid is represented by the first nucleic acid and the second nucleic acid described above.

The binding step and the dissociation step are preferably carried out under the condition of a temperature difference within 40°C, more preferably under the condition of a temperature difference within 30°C, more preferably within 20°C, much more preferably within 10°C, most preferably within 5°C. As shown in each of Examples described later, even when hybridization of the catalytic nucleic acid and the nucleic acid and dissociation of these base pairs are carried out under a substantially constant temperature condition within a temperature difference of about 2°C, the nucleic acid was highly effectively detected.

Incidentally, the operating temperature of the binding step and the operating temperature of the dissociation step do not contain the temperature upon starting the binding step or dissociation step, the temperature at the stage of heating and cooling to the target reaction temperature, upon completion of the binding step or dissociation step, the temperature other than the intended reaction temperature is raised to a temperature related to heating and cooling, and other heating and cooling temperature related to interruption of reaction process and the like. For example, in Example 3 described later, the operating temperature of the binding step and the dissociation step are both 50°C.

The temperature range in which the isothermal reaction is carried out is preferably the temperature change from the start to the end of the reaction step is within the range of 20°C before and after the Tm value of the nucleic acid capable of binding to the catalytic nucleic acid, more preferably within the range of 15°C before and after the Tm value, much more preferably within the range of 10°C before and after the Tm value, still more preferably within the range of 5°C before and after the Tm value, and most preferably within the range of 2°C before and after the Tm value.

A nucleic acid capable of binding to a catalytic nucleic acid is represented by the first nucleic acid and the second nucleic acid described above.

When a plurality of nucleic acids exist to bind to a catalytic nucleic acid in the reaction step, the average value of the Tm values of these nucleic acids is used as a reference. The nucleic acid binding to the catalytic nucleic acid according to the reaction step includes the first nucleic acid and the second nucleic acid of the above-described MNAzyme example.

There are several methods for calculating the Tm value, however, in the present invention and this specification, the nearest neighbor method is adopted.

Incidentally, this reaction step does not include heating and cooling to the target reaction temperature at the start of the reaction step, heating and cooling to a temperature other than the target reaction temperature upon completion of the reaction step, and the other heating and cooling related to interruption and the like. For example, in Example 3 described later, the operation temperature from the start to the end of the reaction process is 50°C.

The temperature range in which the isothermal reaction is carried out is that the temperature change from the start to the end of the reaction step is carried out in the reaction step in the absence of the cationic comb-type polymer, the optimum temperature at which the reaction rate is highest may be set as a reference.

In this case, the temperature range meaning isothermal temperature is preferably within the range of 20°C before and after the optimum temperature, more preferably within the range of 15°C before and after the optimum temperature, much more preferably within 10°C before and after the optimum temperature, and most preferably within the range of 5°C before and after the optimum temperature.

A nucleic acid capable of binding to a catalytic nucleic acid is represented by the first nucleic acid and the second nucleic acid described above.

In determining the optimum temperature, the reaction step may be carried out in the absence of a cationic comb-type polymer and the temperature at which the production rate of the first nucleic acid product is highest may be determined. For example, according to the results obtained in Example 2 described later that the optimum temperature is estimated to be higher than 45°C and lower than 55°C. In determining the optimum temperature, it is preferable to adopt a value specified within 5°C as the estimated value of the optimum temperature.

In addition, the temperature range in which the isothermal reaction may be determined depends on the temperature change from the start to the end of the reaction step in consideration of the sequence and the length of the base hybridizing with the catalytic nucleic acid, the temperature range in which the catalytic nucleic acid to be used can substantially function. Normally, as a nucleic acid substrate or a target nucleic acid of about 15 to 35 bases, the change in temperature from the start to the end of the reaction step is carried out within a temperature range of 20°C to 70°C. When the temperature range is 20°C or higher, hybridization between the catalytic nucleic acid and the nucleic acid is easy to dissociated, therefore, it is preferable in view of turnover promotion. When the temperature range is 70°C or lower, the catalytic nucleic acid and the nucleic acid are more likely to hybridize, therefore, it is preferable in view of turnover promotion.

A good reaction rate is achieved as a practical level as long as it is within a temperature range of 20°C to 70°C. In addition, as shown in Example 2 described later, since the balance between hybridization and dissociation of catalytic nucleic acid and nucleic acid is the best in the vicinity of 50°C, the reaction step is preferably carried out at a temperature range of 30°C to 60°C, more preferably within a temperature range of 40°C to 60°C, and even more preferably in a range of 40°C to 55°C.

In the reaction step, temperature control is not preferably performed in order to hybridize nucleic acids hybridizing with the catalytic nucleic acid and to dissociate these base pairs. As an example of conducting temperature control, temperature control by a commercially available PCR device or the like can be included. As an example of not carrying out the temperature control, leaving the reaction solution at room temperature, or leaving the reaction solution in the incubator kept at the above-mentioned isotherm may be included.

In the method for detecting a nucleic acid of the present invention, the addition of a cationic comb-type copolymer to the reaction system widens the more active temperature range, so there is an advantage that it is not necessary to precisely control the reaction temperature.

### (Nucleic acid)

The first nucleic acid or the second nucleic acid to be detected may be natural or artificially synthesized. The first nucleic acid is not particularly limited as long as it can serve as a substrate for the catalytic nucleic acid used in the reaction step. As the first nucleic acid or the second nucleic acid, for example, DNA, methylated DNA, alkylated DNA, RNA, microRNA, siRNA, mRNA, tRNA, snoRNA, noncoding RNA or a derivative thereof, amplicon or complex thereof may be included.

The origin of the first nucleic acid or the second nucleic acid to be detected is not particularly limited, however, it is preferably derived from peripheral blood, serum, plasma, saliva, oral swab, FFPE (paraffin embedded) section or tissue.

In the nucleic acid detection method of the present invention, nucleic acids derived from cancer-related genes, genetic diseases, genes related to drug metabolism and efficacy, viral genes, and bacterial genes can be included as target nucleic acids.

Examples of cancer-related genes include k-ras gene, BRAF gene, PTEN gene, ALK gene, EGFR gene, N-ras gene, p53 gene, BRCA1 gene, BRCA2 gene, or APC gene, ABL, ALK and the like. Genes related to genetic diseases include genes reported to be associated with various congenital metabolic disorders and the like.

In recent years, it has been found that the above-mentioned cancer gene and the like are circulating in the peripheral blood, and by detecting them, it is possible to predict drug resistance, exacerbation and recurrence of cancer, and it is known that it will be an index for switching treatment. Therefore, it is reported that the circulating DNA is cell free, and it is reported that it is fragmented roughly 100 bp or less, and this method can be adapted to detect those DNAs.

In addition, tumor cells and exosomes are included in the peripheral blood of cancer patients in addition to circulating DNA derived from tumors. It is reported that exosomes contain abundant microRNAs and by detecting them, it also leads to the early detection of cancer. According to the nucleic acid detection method of the present invention, such a microRNA can be directly detected without including a nucleic acid amplification step.

Regarding metabolism of drugs, genes such as cytochrome P450 and transporter, which are enzymes involved in drug metabolism, can be included. Viral genes and bacterial genes include genes such as hepatitis C virus and hepatitis B virus. Furthermore, HLA, which is a human leukocyte antigen gene not directly related to the cause of the disease, is also important in relation to compatibility of transplantation and side effects of drugs. In addition, genetic mutations encoded by mitochondria have been suggested to be associated with diseases, and mutations in these genes can also be targets.

The sample of the present invention may be prepared by using a nucleic acid amplification reaction, if necessary. As an indication, when the number of nucleic acids to be detected contained in the sample is less than 10⁶ copies, a nucleic acid amplification step may be included. As a nucleic acid amplification method, it can be appropriately selected from well-known nucleic acid amplification reactions such as PCR method, LCR method (Ligase Chain Reaction), 3SR method (Self-sustained Sequence Replication), SDA method (Strand Displacement Amplification). Among these amplification methods, the PCR method can be used for the present invention since the target nucleic acid fragment can be obtained as a double-stranded amplification product. In addition, nucleic acid substrate can also be prepared by using gene amplification reaction, but chemical synthesis is optimal considering freedom of introduction position of labeling substance and the like. Also, if the target nucleic acid is 10⁶ copies or more, it can be detected even without a nucleic acid amplification step. Furthermore, the target nucleic acid can be developed not only for DNA but also for detection of RNA and the like. For example, direct detection such as miRNA is also possibly detected.

Furthermore, combining the present invention with techniques such as "Microchamber used for detection of single-molecule enzyme activity", Japanese Patent No. 3727026 (October 7, 2005) / Japanese Patent Application No. 2003-106098 (Apr. 10, 2003) and "A method of enclosing beads, a method of detecting a molecule, an array, a kit and a target molecule detection device", Japanese Patent Application No. 2011-50629 (March 8, 2011) enables to reach a detection from the single target nucleic acid utilizing a catalytic nucleic acid, it is presumed that the time to detect a target nucleic acid can be greatly shortened without carrying out a nucleic acid amplification step in order to carry out a reaction in a fine space.

### « Quantitative Method of Nucleic Acid »

In the method of quantifying a nucleic acid of the present invention, the amount of the nucleic acid to be detected is determined by measuring the amount of the first nucleic acid product detected and/or the amount of unreacted first nucleic acids.

The nucleic acid to be detected may be the first nucleic acid itself as a substrate or may be another nucleic acid as another detection target through detection of the first nucleic acid. As the other nucleic acid, the second nucleic acid described above can be included. The amount of the nucleic acid can be determined by comparing the result obtained in the reaction step according to the present invention to a known sample whose amount of the nucleic acid is known, for examples, a conventional method such as calculating the amount based on a calibration curve prepared in advance and the like can be used.

### <Method of identifying base sequence of nucleic acid>

The method for identifying a base sequence of a nucleic acid of the present invention including a reaction step to obtain a first nucleic acid product by hybridizing a first nucleic acid which is a substrate to a first binding site of a catalytic nucleic acid equipped with an active site having a catalytic action; a detection step for detecting the first nucleic acid products and/or unreacted first nucleic acids; and an identification step for identifying the base sequence of nucleic acid based on the measurement results obtained by the detection step, wherein the hybridization is carried out in the presence of a cationic comb-type polymer.

As an example of "the hybridization is carried out in the presence of a cationic comb-type polymer", it may be "the hybridization is carried out in a solution to which a cationic comb-type polymer is added".

The method for identifying a base sequence of a nucleic acid of the present invention, the catalytic nucleic acid further comprises a second binding site which is capable of being hybridized to a second nucleic acid, the second nucleic acid is hybridized with the second binding site so that the catalytic action of the catalytic nucleic acid in the reaction steps is enabled. The detection step may include detecting the second nucleic acid by detecting the first nucleic acid products and/or unreacted first nucleic acids.

The method for identifying a base sequence of a nucleic acid in the specification of the present application identifies whether the nucleic acid in the sample is the nucleic acid containing the same base sequence as the target base sequence by detecting whether the first nucleic acid to be a substrate of the catalytic nucleic acid is reacted to the catalytic nucleic acid.

When a nucleic acid chain having a similar base sequence exists in the reaction system, a nucleic acid chain having a base sequence completely complementary to the first binding site of the catalytic nucleic acid preferentially binds to the first binding site. Similarly, when the catalytic nucleic acid has a second binding site, a nucleic acid chain having a completely complementary base sequence to the second binding site preferentially binds to the second binding site. This difference in binding causes a difference in the amount of production of the first nucleic acid products and/or unreacted first nucleic acids, and the base sequence of the nucleic acid can be identified based on the measurement result obtained in the detection step.

As described above, the nucleic acid base sequence identification method is capable of identifying whether the nucleic acid contained in the sample is a nucleic acid containing the same base sequence as the target base sequence by knowing the sequence of the first binding site of the catalytic nucleic acid.

The reaction step and the detection step are common to those described in the nucleic acid detection method described above, so the explanation is omitted.

The identification method of the present invention can be suitably used for identifying mutations or polymorphisms in the base sequence of a nucleic acid to be detected. Specifically, the base sequence of the region containing the mutation site of the gene mutation is considered as the target base sequence. In the present invention, a gene mutation means a difference in the base sequence of a gene existing among individuals of the same species, and the mutation site means a different site in the base sequence. Specifically, base sequence differences arise due to substitution, deletion, or insertion of one or more bases in the base sequence. Examples of such gene mutations include SNPs and CNV polymorphisms. In addition, the term "gene mutation" in the present invention includes acquired mutations such as somatic mutations, which are differences in the base sequence of genes present among cells in the same individual in addition to the congenital mutation such as gene polymorphism such as SNP.

In the identification method of the present invention, the target gene mutation or polymorphism is not particularly limited, and mutation or polymorphism possessed by the gene exemplified in the above-mentioned "nucleic acid detection method" can be similarly exemplified.

The method for identifying a base sequence of a nucleic acid of the present invention may identify a mutation or a polymorphism in a base sequence of a nucleic acid to be detected by comparing the amount of the first nucleic acid product and unreacted first nucleic acids. The first nucleic acid product and unreacted first nucleic acid to be compared may be, for example, the first nucleic acid product and unreacted first nucleic acid present in the same reaction system through the reaction step, or the first nucleic acid product and unreacted first nucleic acid present in the different reaction system through the same reaction step. When comparing the amounts of the first nucleic acid product and unreacted first nucleic acid present in the same reaction system, it is possible to exemplify a case of identifying between a heterozygote and a homozygote of a gene polymorphism.

### «Nucleic acid detection kit»

A kit for nucleic acid detection of the present invention is a kit used for a method for detecting a nucleic acid of the present invention, and is characterized by including a cationic comb-type polymer. In addition, the nucleic acid detection kit includes a catalytic nucleic acid having a first binding site capable of hybridizing with a sequence of a first nucleic acid which can be a substrate of a catalytic nucleic acid. In addition to the first binding site, a catalytic nucleic acid having a second binding site of hybridizing with a second nucleic acid, a buffer, a cation or a reagent added to the reaction of the catalytic nucleic acid such as an organic solvent, and a reagent for detecting a label of a labeling substance, or the like can be combined. In this manner, by kitizing reagents and the like necessary for the identification method of the present invention, it is possible to identify the target base sequence more simply and in a short time.

The kit for nucleic acid detection of the present invention is used as a kit for identifying a target base sequence by including a catalytic nucleic acid having a base sequence homologous to a target base sequence desired to be identified.

The kit for detecting a nucleic acid of the present invention is used for a kit for identifying the target base sequence so as to detect the gene mutation or polymorphism by including the catalytic nucleic acid having the base sequence homologous to a region containing a mutation site of a specific genetic type of a gene mutation.

### «Reaction chip»

The reaction chip of the present invention is a reaction chip used for the method for detecting a nucleic acid of the present invention, and the reaction chip holds a cationic comb-type copolymer and/or a catalytic nucleic acid in advance, and the chip includes reaction wells for storing a cationic comb-type copolymer and a catalytic nucleic acid.

The reaction well can be used as a reaction chamber for carrying out the reaction step in the nucleic acid detection method of the present invention by reacting a reaction solution containing a cationic comb-type copolymer, a catalytic nucleic acid and a nucleic acid substrate. In the reaction chip, the detection step in the nucleic acid detection method of the present invention may be carried out.

In the field of biochemical reactions such as DNA reactions, protein reactions and the like, a technique called µ-TAS (Total Analysis System) or Lab-on-Chip is known as a reaction device for treating a trace amount of a sample solution. This reaction device comprises one chip and a cartridge provided with a plurality of wells and flow passages, and it is possible to analyze a plurality of specimens or to perform a plurality of reactions. These techniques have been considered to have various merits because it is possible to reduce the amount of chemicals handled by downsizing the chips and cartridges.

In the reaction chip of the present invention, the cationic comb-type copolymer and the catalytic nucleic acid may be held in advance in the reaction well, or one or both of the cationic comb-type copolymer and the catalytic nucleic acid may be held at a part different from the well, and may be held in a part inside the reaction chip of the present invention. In this case of carrying out the reaction step, one or both of the cationic comb-type copolymer and the catalytic nucleic acid is distributed from the holding part to the reaction well. Therefore, the reaction chip of the present invention preferably has a flow passage for feeding to the reaction well, and further includes a holding part for holding the cationic comb-type copolymer or the catalytic nucleic acid. A plurality of holding parts may be provided, and in addition to the cationic comb-type copolymer, the catalytic nucleic acid, other reagents essential for the reaction step may be held. Each of the reagents may be held one by one in separate holding parts, or plural kinds of reagents may be held in the same holding part. In the method for detecting a nucleic acid of the present invention, for example, since the reaction step can be carried out under isothermal conditions at room temperature, only performing the step of distributing the reagents and samples essential for the reaction step to the reaction well, and it is also possible to start and continue the reaction process.

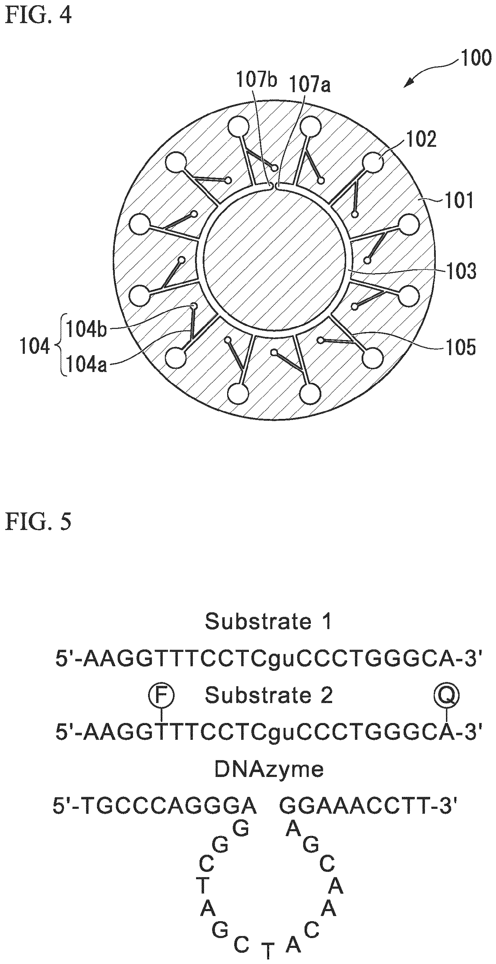

FIG. 4 is a plan view showing one embodiment of the reaction chip according to the first aspect of the present invention.

The reaction chip 100 has a plurality of reaction wells 102 on the base material 101 and a flow passage for feeding a solution, for example, a liquid sample (solution) to the reaction well 102. The flow passage has one main flow passage 103 communicating with at least each reaction well and has a side passage 105 connecting the main flow passage and the reaction well in order to feed to each reaction well.

The reaction chip 100 has an injection port for injecting a solution. In the embodiment of FIG. 4, an inlet (INLET) 107a is provided at an end portion of the main flow passage 103 and an outlet (OUTLET) 107b of an excess solution which also serves as an air escape port at the other end portion. Furthermore, in the sample analysis chip of the present embodiment, in the side passage 105 that connects each reaction well 102 and the main flow passage 103, the holding part 104 is provided for each side passage.

The holding part 104 can be constituted by a branch flow passage 104a branching from the side passage and a chamber 104b connected to the branch flow passage.

In the reaction chip of this embodiment, a cationic comb-type copolymer, a nucleic acid substrate (first nucleic acid), and a reaction buffer are preliminarily held in the reaction well 102, and a solution containing catalytic nucleic acid is held in the holding part 104. When the reaction chip is rotated after injecting the sample liquid containing the nucleic acid to be detected into the injection port (INLET), the solution containing the catalytic nucleic acid and the sample solution are fed into each well 102 by centrifugal force generated by rotation, then, the reaction is initiated in the reaction well 102.

### EXAMPLE

Next, the present invention will be described in more detail by showing examples, but the present invention is not limited to the following examples.

### [Example 1-1] Single turnover

In order to confirm that the cationic comb-type copolymer accelerated and promoted turnover with substrate and catalytic nucleic acid, the experiment was carried out by the following procedure.

FIG. 5 shows the sequence of the DNAzyme (SEQ ID NO: 1) used in this example and the sequence (SEQ ID NO: 2) of the nucleic acid substrate and the nucleic acid (first nucleic acid) which is the target nucleic acid to be detected. The base sequence shown in lower case letters in the sequence indicates that the sequence portion is RNA. As a nucleic acid substrate, Substrate 1 which is not fluorescently labeled was used.

A reaction solution containing a DNAzyme, a nucleic acid substrate (also a target nucleic acid in this example), and a cationic comb-type copolymer was prepared (PLL-g-Dex (+)). The composition (final concentration) of the solution is shown below.

HEPES (pH = 7.3) Buffer solution 50 mM
NaCl 150 mM
MgSO₄ 0.5 mM
Nucleic acid substrate 500 nM
DNAzyme 750 nM
PLL-g-Dex (cationic comb-type polymer)

The cationic comb-type polymer is obtained by polymerizing dextran in the side chain with polylysine as the main chain, the molecular weight of polylysine is about 7500, the molecular weight of dextran is about 9000, and the dextran content is 90 wt%. Regarding the concentration, the cationic comb-type polymer was added so that the amino group (cationic comb-type polymer) relative to the phosphate group (DNA) becomes 2 based on the total DNA in the reaction solution. The charge of cationic comb-type polymer to the charge of nucleic acid is 2 in reaction solution. The above-mentioned values for the charge ratio of the phosphate group and the nucleic acid were based on the phosphate groups of all the DNAs in the reaction solution, and the DNA was calculated including both the DNAzyme and the nucleic acid substrate.

DNAzyme and substrate were added to be DNAzyme: substrace =1 : 1.5 (mol) in the reaction solution. In the reaction solution, the magnesium was added so that the final concentration of the magnesium was 0.5 mM.

As a control, a reaction solution containing no cationic comb-type copolymer in the composition of the above solution was prepared (PLL-g-Dex (-)).

PLL-g-Dex (+) and PLL-g-Dex (-) were incubated at 25°C. The reaction was stopped by adding EDTA. The sample after the reaction was applied to 16% polyacrylamide gel electrophoresis.

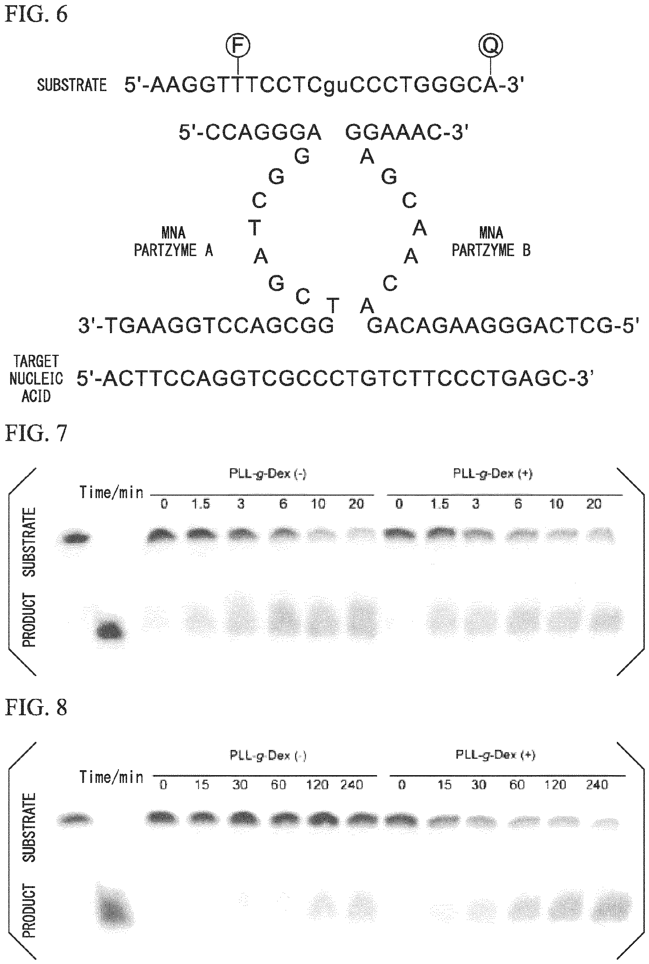

The results are shown in Fig. 7. In PLL-g-Dex (+) to which a cationic comb-type polymer was added, as compared with PLL-g-Dex (-) to which a cationic comb-type polymer was not added, the rate at which the nucleic acid substrate was cleaved and the nucleic acid product was produced was fast.

### [Example 1-2] Multiple turnover

In order to confirm that the cationic comb-type copolymer accelerated and promoted turnover with substrate and catalytic nucleic acid, the experiment was carried out by the following procedure.

The sequences of the DNAzyme and the nucleic acid substrate (first nucleic acid) used in this example were the same as those used in the above [Example 1-1].

A reaction solution containing a DNAzyme, a nucleic acid substrate (which is also a target nucleic acid in this example), and a cationic comb-type polymer was prepared (PLL-g-Dex (+)). The composition (final concentration) of the solution is shown below.

HEPES (pH = 7.3) Buffer solution 50 mM
NaCl 150 mM
MgSO₄ 0.5 mM
Nucleic acid substrate 500 nM
DNAzyme 16.7 nM
PLL-g-Dex (cationic comb-type polymer)

In the reaction solution, the cationic comb-type polymer and the concentration thereof are the same as in [Example 1-1]. In the reaction solution, DNAzyme and substrate were added to be DNAzyme: Substrate = 1: 30 (mol), and the magnesium was added so that the final concentration of the magnesium was 0.5 mM.

As a control, a reaction solution containing no cationic comb-type copolymer in the composition of the above solution was prepared (PLL-g-Dex (-)).

PLL-g-Dex (+) and PLL-g-Dex (-) were incubated at 25°C. The reaction was stopped by adding EDTA. The sample after the reaction was applied to 16% polyacrylamide gel electrophoresis.

The results are shown in Fig. 8. For PLL-g-Dex (+) to which a cationic comb-type polymer was added, a band of the cleavage was confirmed from about 15 minutes after the reaction. On the other hand, in PLL-g-Dex (-) to which no cationic comb-type polymer was added, it took more than 60 minutes for the band of the nucleic acid substrate product to be confirmed. In addition, it can be seen that the cleavage reaction efficiency is poor because the obtained band is thin.

According to the result of the above [Example 1-1] and [Example 1-2], it was suggested that the interaction between cationic comb-type copolymer and DNA promotes and accelerates turnover of DNAzyme more than ever before while maintaining the catalytic activity of DNAzyme.

### [Example 2]

In order to confirm whether the cationic comb-type copolymer activates the DNAzyme, promotes turnover, and widens the adaptation temperature range, the experiment was conducted according to the following procedure.

The sequences of the DNAzyme and the nucleic acid substrate (first nucleic acid) used in this example are the same as those used in the above [Example 1-1].

Substrate 2 which was labeled with fluorescein (F) and black hole quencher I (Q) was used as the nucleic acid substrate (FIG. 5).

A reaction solution containing a DNAzyme, a nucleic acid substrate (which is also a target nucleic acid in this example), and a cationic comb-type polymer was prepared (PLL-g-Dex (+)). The composition (final concentration) of the solution is shown below.
HEPES (pH = 7.3) Buffer solution 50 mM
NaCl 150 mM
MnCl₂ 0.5 mM
Nucleic acid substrate 200 nM
DNAzyme 6.7 nM
PLL-g-Dex (cationic comb-type polymer)

The cationic comb-type polymer is obtained by polymerizing dextran in the side chain with polylysine as the main chain, the molecular weight of polylysine is about 7500, the molecular weight of dextran is about 9000, and the dextran content is 90 wt%. Regarding the concentration, the cationic comb-type polymer was added so that the amino group (cationic comb-type polymer) relative to the phosphate group (DNA) becomes 2 based on the total DNA in the reaction solution. The charge of cationic comb-type polymer to the charge of nucleic acid is 2 in reaction solution. The above-mentioned values for the charge ratio of the phosphate group and the nucleic acid were based on the phosphate groups of all the DNAs in the reaction solution, and the DNA was calculated including both the DNAzyme and the nucleic acid substrate.

As a control, a reaction solution containing no cationic comb-type copolymer in the composition of the above solution was prepared (PLL-g-Dex (-)).

PLL-g-Dex (+) were respectively incubated at 18°C to 75°C (18°C, 25°C, 37°C, 45°C, 50°C, 55°C, 60°C, 65°C, 70°C, and 75°C). PLL-g-Dex (-) were respectively incubated at 25°C to 65°C (25°C, 37°C, 45°C, 50°C, 55°C, 60°C, and 65°C). The fluorescence associated with cleavage of the nucleic acid substrate at each temperature was measured using a fluorescence spectrophotometer (manufactured by JASCO Corporation).

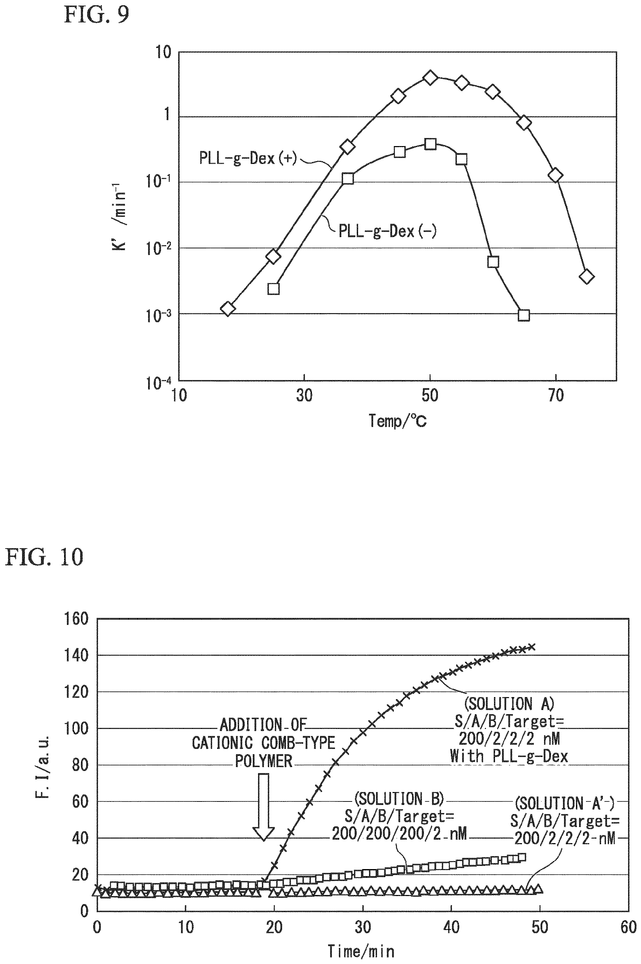

The results are shown in Fig. 9. The vertical axis shows the rate constant and the horizontal axis shows the reaction temperature. From the results shown in FIG. 9, in the system without adding a cationic comb-type copolymer, the range of the temperature showing the maximum value of the reaction rate is small (PLL-g-Dex (-)), however addition of the cationic comb-type copolymer increased the reaction rate at each reaction temperature and broadened out the adaptation temperature range (PLL-g-Dex (+)).

Therefore, by promoting dissociation / re-hybridizing of the nucleobase pair, the cationic comb-type copolymer brings the thermodynamically most stable state from the metastable mis-hybrid to the most stable state, thus resulting in activating the DNAzyme and promoting turnover, and widening the adaptation temperature range.

### [Example 3]

In order to confirm that the cationic comb-type copolymer significantly increases the reactivity of the MNAzyme, the following experiment was conducted.

The sequence of the nucleic acid substrate (SEQ ID NO: 2), the sequence of MNA partzyme A (SEQ ID NO: 3), the sequence of MNA partzyme B (SEQ ID NO: 4), the sequence of the target nucleic acid (SEQ ID NO: 5) are shown in FIG.6. As shown in FIG. 6, a nucleic acid substrate which was labeled with fluorescein (F) and black hole quencher I (Q) was used.

First, reaction solutions A, A' and B containing MNAzyme partzyme, target nucleic acid and nucleic acid substrate were prepared with the following compositions.
HEPES (pH = 7.3) Buffer solution 50 mM
NaCl 150 mM
MnCl₂ 0.5 mM
Nucleic acid substrate 200 nM
Partzyme A of MNAzyme A predetermined concentration as follows.
Partzyme B of MNAzyme A predetermined concentration as follows.
Target nucleic acid 2 nM (final concentration)

### Solution A, A'

S/A/B/Target = 200/2/2/2 (All units are nM)

### Solution B

S/A/B/Target = 200/200/200/2 (All units are nM)
(S is substrate, A is partzyme A, B is partzyme B, and Target is a target nucleic acid.)

Solution A, Solution A' and Solution B were incubated at 50°C, and after 18 minutes from the start of incubation, PLL-g-Dex (cationic comb-type polymer) was added to the solution A so that the amino group (cationic comb-type polymer) relative to the phosphate group (DNA) becomes 2. Charge of cationic comb-type relative to a charge of nucleic acid is 2 in the reaction solution. The above values for the charge ratio of the phosphate group and the nucleic acid were based on the phosphate groups of all the DNAs in the reaction solution and the DNA was calculated including both the MNAzyme and the nucleic acid substrate.

The cationic comb-type polymer is obtained by polymerizing dextran in the side chain with polylysine as the main chain, the molecular weight of polylysine is about 7500, the molecular weight of dextran is about 9000, and the dextran content is 90 wt%. The fluorescence associated with cleavage of the nucleic acid substrate in each reaction solution was measured using fluorescence spectrophotometer V-770 (manufactured by JASCO Corporation)).

The results are shown in Figure 10. Compared with the solution A' in which the cationic comb-type copolymer was not added, the solution A to which the cationic comb-type copolymer was added from the middle of the reaction, the fluorescence signal associated with the cleavage reaction increased at the time when the cationic comb-type copolymer was added. Especially, in the solution A, the fluorescence signal associated with the cleavage reaction dramatically increases when the cationic comb-type copolymer was added.

By adding a predetermined concentration of cationic polymer, accumulation of signal due to cleavage was observed in the solution A even when the final concentration of MNAzyme is 2 nM. On the other hand, the solution B which is a reaction system with no polymer addition, fluorescence signal due to substrate cleavage reaction was hardly observed even when MNAzyme was increased to 200 nM. This indicates that the cationic comb-type copolymer markedly promotes the reaction by MNAzyme in an isothermal reaction at 50°C.

### [Example 4-1]

In order to confirm that the cationic comb-type copolymer significantly increases the reactivity of MNAzyme, the following experiment was conducted.

The nucleic acid substrate, MNA partzyme A, MNA partzyme B, and target nucleic acid used in this example are the same as those used in the above [Example 3].

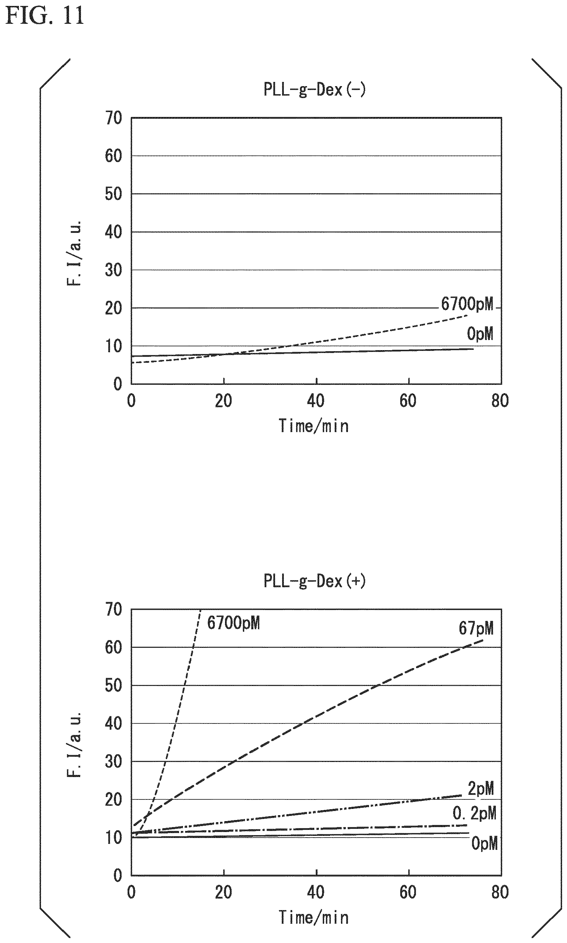

A reaction solution containing a partzyme of MNAzyme, a target nucleic acid, a nucleic acid substrate, and a cationic comb-type polymer was prepared in the following composition (PLL-g-Dex (+)). The concentration of the target nucleic acid was added at the concentration shown in the graph of FIG. 11.
HEPES (pH = 7.3) Buffer solution 50 mM
NaCl 150 mM
MnCl₂ 0.5 mM
Nucleic acid substrate 200 nM
Partzyme A of MNAzyme 6.7 nM
Partzyme B of MNAzyme 6.7 nM
Target nucleic acid Predetermined concentration shown in FIG. 11
PLL-g-Dex (cationic comb-type polymer)

The cationic comb-type polymer is obtained by polymerizing dextran in the side chain with polylysine as the main chain, the molecular weight of polylysine is about 7500, the molecular weight of dextran is about 9000, and the dextran content is 90 wt%. The cationic comb-type polymer was added so that the concentration in which an amino group (cationic comb-type polymer) relative to a phosphate group (DNA) becomes 2.

As a control, a reaction solution containing no cationic comb-type polymer in the composition of the solution was prepared (PLL-g-Dex (-)).

PLL-g-Dex (+) and PLL-g-Dex (-) were incubated at 37°C. The fluorescence associated with cleavage of the nucleic acid substrate in each reaction solution was measured using a fluorescence spectrophotometer FP-8300 (manufactured by JASCO Corporation).

The results are shown in FIG. 11. FIG. 11 is a graph showing the cleavage efficiency of the substrate of MNAzyme depending on the presence or absence of a cationic comb-type copolymer at each target nucleic acid concentration. The fluorescence when the target nucleic acid concentration was 6700 pM in the reaction not containing the cationic comb-type polymer and the fluorescence when using the target nucleic acid concentration of 2 pM in the reaction containing the cationic comb-type polymer were almost equivalent. From this, it was found that the cationic comb-type copolymer promoted the reaction of MNAzyme, and it enabled to reduce the concentration of the target nucleic acid as thin as about 3000 times as compared with the case without the cationic comb-type copolymer.

### [Example 4-2]

In order to confirm that the cationic comb-type copolymer significantly increases the reactivity of MNAzyme, the following experiment was conducted.

The nucleic acid substrate, MNA partzyme A, MNA partzyme B, and target nucleic acid used in this example are the same as those used in the above [Example 4-1].

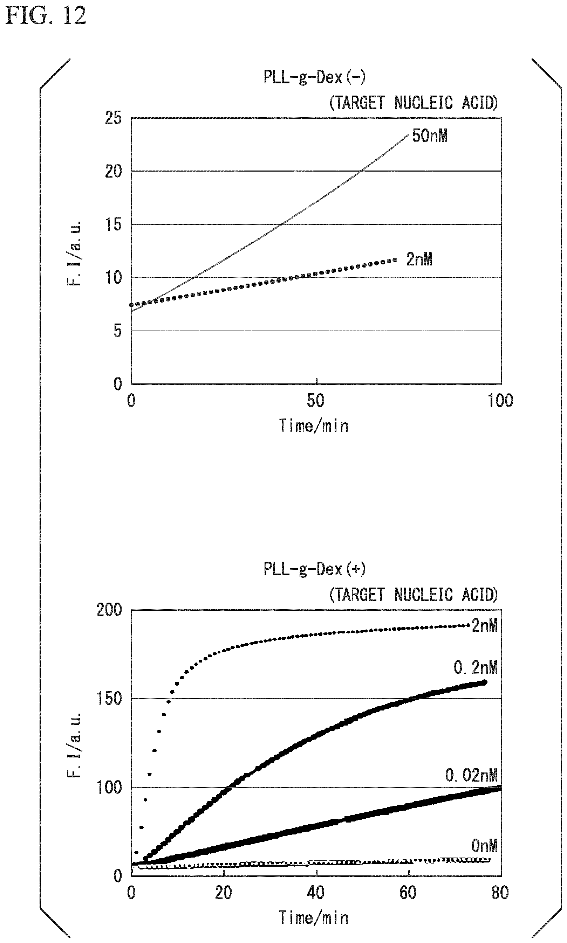

A reaction solution containing a partzyme of MNAzyme, a target nucleic acid, a nucleic acid substrate, and a cationic comb-type polymer was prepared in the following composition (PLL-g-Dex (+)). The concentration of the target nucleic acid was added at the concentration shown in the graph in FIG. 12.
HEPES (pH = 7.3) Buffer solution 50 mM
NaCl 150 mM
MnCl₂ 0.5 mM
Nucleic acid substrate 200 nM
Partzyme A 2 nM
Partzyme B 2 nM
Target nucleic acid Predetermined concentration shown in FIG. 12
PLL-g-Dex (cationic comb-type polymer)

The cationic comb-type polymer is obtained by polymerizing dextran in the side chain with polylysine as the main chain, the molecular weight of polylysine is about 7500, the molecular weight of dextran is about 9000, and the dextran content is 90 wt%. Regarding the concentration, the cationic comb-type polymer was added so that the concentration in which an amino group (cationic comb-type polymer) relative to a phosphate group (DNA) becomes 2. Charge of cationic comb-type relative to a charge of nucleic acid is 2 in the reaction solution. The above values for the charge ratio of the phosphate group and the nucleic acid were based on the phosphate groups of all the DNAs in the reaction solution and the DNA was calculated including both the MNAzyme and the nucleic acid substrate.

As a control experiment, a reaction solution containing no cationic comb-type copolymer in the composition of the above solution was prepared (PLL-g-Dex (-)).

PLL-g-Dex (+) and PLL-g-Dex (-) were incubated at 50°C. The fluorescence associated with cleavage of the nucleic acid substrate in each reaction solution was measured using a fluorescence spectrophotometer (manufactured by JASCO Corporation).

The results are shown in Fig. 12. FIG. 12 is a graph showing the cleavage efficiency of MNAzyme substrate with or without cationic comb-type copolymer at each target nucleic acid concentration. The fluorescence when a target nucleic acid concentration of 0.02 nM was used in the reaction containing a cationic comb-type polymer was greater compared to the fluorescence when a target nucleic acid concentration of 50 nM was used in the reaction not containing a cationic comb-type polymer. From this, it was found that the cationic comb-type copolymer promoted the reaction of MNAzyme, and it enabled to reduce the concentration of the target nucleic acid as thin as about 1000 times as compared with the case without the cationic comb-type copolymer.

According to the results of Examples [4-1] and [4-2] above, adding a cationic comb-type copolymer to the reaction system were capable of extending the catalytic activity adaptation temperature range of the MNA complex, promoting the turnover of MNA complex formation, and stabilizing the MNA complex and substrate complex than conventional methods, and it enabled to detect the presence of about 10⁶ copies of template nucleic acid at a constant temperature of 37°C and 50°C by adding a cationic comb-type copolymer to the reaction system.

In the case of using a system in which no conventional cationic comb-type copolymer is present, when a signal with an S/N ratio of 2 or less is obtained by a reaction at a constant temperature of 37°C for 70 minutes, the detection limit of the target nucleic acid is 70nM. However, in the case of adding a cationic comb-type copolymer, it is capable of obtaining a signal with an S/N ratio of about 7 in about 20 minutes, and it enables to detect the target nucleic acid of 2 pM in about 70 minutes. Therefore, the detection sensitivity of the target nucleic acid was improved by about 35,000 times, suggesting that if the required number of copies of the target nucleic acid is about 1.2 × 10⁶, it could be directly detected without the nucleic acid amplification step.

The target nucleic acid of 2 pM contains approximately 10⁶ copies, and the concentration of miRNA to be detected contained in 1 L of serum or plasma may be 10⁸ to 10¹⁰ (0.2 to 2.0 fM) in some cases. Therefore, even when such miRNA concentration is used as a target nucleic acid, the method for detecting a nucleic acid of the present invention may possibly able to detect the nucleic acid, without conducting a nucleic acid amplification step such as PCR.

Although the embodiments of the present invention have been described in detail with reference to the drawings, the specific configuration is not limited to these embodiments, and various modifications such as design changes and the like may be made without departing from the scope of the present invention.

### INDUSTRIAL APPLICABILITY

The method of detecting a target base sequence of the present invention can greatly reduce the cost as it can be identified with high accuracy and quickly from the target nucleic acid in the sample without going through the nucleic acid amplification step. The method can be applied in the field of clinical examination, particularly in somatic mutations and the like.

### BRIEF DESCRIPTION OF THE REFERENCE SYMBOLS

100 ...· Reaction chip
101 ... Base material
102 ...· Reaction wells
103 ... Main flow passage
104 ... Holding part
104a ... Branch flow passage
104b ... Chamber
105 ... Side passage
107a ... INLET
107b ... OUTLET

## Claims

1. A method for detecting a nucleic acid comprising:
a reaction step to obtain a first nucleic acid product by hybridizing a first nucleic acid which is a substrate to a first binding site of a catalytic nucleic acid equipped with an active site having a catalytic action; and
a detection step for detecting at least one of the first nucleic acid product or a unreacted first nucleic acid,
wherein, the hybridization is carried out in the presence of a cationic comb-type polymer.

2. The method for detecting a nucleic acid according to claim 1, further comprising a step for dissociating hybridization between the first nucleic acid product hybridized to the first binding site of the catalytic nucleic acid in the reaction step.

3. The method for detecting a nucleic acid according to claim 1 or 2, wherein the catalytic nucleic acid further equips a second binding site capable of hybridizing to a second nucleic acid,
hybridizing the second nucleic acid to the second binding site causes the catalytic action of the catalytic nucleic acid in the reaction step, and
detecting the second nucleic acid by detecting at least one of the first nucleic acid product or unreacted first nucleic acids in the detection step.

4. The method for detecting a nucleic acid according to any one of claims 1 to 3, wherein the cationic comb-type copolymer having a main chain which is a polymer chain containing a cationic group and a side chain which is a hydrophilic group.

5. The method for detecting a nucleic acid according to claim 4, wherein the main chain of the cationic comb-type copolymer is polylysine.

6. The method for detecting a nucleic acid according to claim 4 or 5, wherein the side chain of the cationic comb-type copolymer is dextran.

7. The method for detecting a nucleic acid according to any one of claims 4 to 6, wherein the molecular weight of the main chain portion of the cationic comb-type copolymer is 5,000 or more.

8. The method for detecting a nucleic acid according to any one of claims 1 to 7, wherein the reaction step is carried out in the presence of a divalent metal ion.

9. The method for detecting a nucleic acid according to claim 8, wherein the divalent metal ion is magnesium ion or manganese ion.

10. The method for detecting a nucleic acid according to any one of claims 2 to 9,
a step of hybridizing the first nucleic acid to the first binding site,
a step for dissociating hybridization between the first nucleic acid product hybridized to the first binding site of the catalytic nucleic acid and the catalytic nucleic acid,
wherein these steps are conducted under conditions of a temperature difference of within 40°C.

11. The method for detecting a nucleic acid according to any one of claims 1 to 10, wherein the reaction step is carried out within a temperature range of 20°C to 70°C.

12. The method for detecting a nucleic acid according to any one of claims 1 to 11, wherein the first nucleic acid is labeled with a labeling substance.

13. The method for detecting a nucleic acid according to claim 12, wherein the first nucleic acid is labeled with a first labeling substance and a second labeling substance which are different from each other, and transferring energy between the first labeling substance and the second labeling substance.

14. The method for detecting a nucleic acid according to claim 13, wherein the labeling substance is a fluorescent substance.

15. The method for detecting a nucleic acid according to claim 13 or 14, wherein the first labeling substance and the second labeling substance are a combination of a fluorescent substance and a quenching substance.

16. The method for detecting a nucleic acid according to any one of claims 1 to 15, wherein the first nucleic acid or the second nucleic acid to be detected is a nucleic acid at least one selected from a group consisting of DNA, methylated DNA, alkylated DNA, RNA, microRNA, siRNA, mRNA, tRNA, snoRNA, noncoding RNA, the derivative thereof, the amplicon thereof, and the complexes thereof.

17. The method for detecting a nucleic acid according to any one of claims 1 to 16, wherein the first nucleic acid or the second nucleic acid to be detected is derived from peripheral blood, serum, plasma, saliva, buccal swab, FFPE (paraffin embedded) section or tissue.

18. A method for quantifying a nucleic acid **characterized by** measuring the amount of at least one of the first nucleic acid product or unreacted first nucleic acids detected by the nucleic acid detection method according to any one of claims 1 to 17.

19. A method for identifying a base sequence of a nucleic acid, comprising:
a reaction step of obtaining a first nucleic acid product by hybridizing a first nucleic acid which is a substrate to a first binding site of a catalytic nucleic acid equipped with a catalytic active site having a catalytic action;
a detection step of detecting at least one of the first nucleic acid product or unreacted first nucleic acids; and
an identification step of identifying the base sequence of the nucleic acid based on the measurement result obtained in the detection step,
wherein the hybridization is carried out in the presence of a cationic comb-type polymer.

20. The method for identifying a base sequence of a nucleic acid according to claim 19, the catalytic nucleic acid further equips a second binding site capable of hybridizing with the second nucleic acid, hybridizing the second nucleic acid to the second binding site causes the catalytic action of the catalytic nucleic acid in the reaction step, and detecting the second nucleic acid by detecting at least one of the first nucleic acid product or unreacted first nucleic acids in the detection step.

21. A method for identifying a mutation or polymorphism of a nucleic acid, **characterized by** identifying a mutation or polymorphism in a base sequence of a nucleic acid to be detected by the method for identifying a base sequence of a nucleic acid according to claim 19 or 20.

22. The method for identifying a mutation or polymorphism of a nucleic acid according to claim 21, wherein the mutation or polymorphism of a base sequence of a nucleic acid to be detected by comparing the amount of the first nucleic acid product and unreacted first nucleic acids.

23. A kit for detecting a nucleic acid comprising the cationic comb-type polymer, wherein the kit is used for the method for detecting a nucleic acid according to any one of claims 1 to 17.

24. A reaction chip for using the method for detecting a nucleic acid according to any one of claims 1 to 17, wherein the reaction chip preliminary holds at least one of the cationic comb-type copolymer or the catalytic nucleic acid, and the reaction chip includes a reaction well for holding the cationic comb-type copolymer and the catalytic nucleic acid.


### [Sequence List]

SEQUENCE LISTING

<110>  TOPPAN Printing Co., Ltd.
       Tokyo Institute of Technology

<120>  Method for detecting target nucleic acid

<130>  P20140521

<160>  5

<170>  PatentIn version 3.5

<210>  1
<211>  34
<212>  DNA
<213>  Artificial Sequence

<220>
<223>  Description of Artificial Sequence: DNAzyme

<400>  1
tgcccaggga ggctagctac aacgaggaaa cctt                                    34

<210>  2
<211>  22
<212>  DNA
<213>  Artificial Sequence

<220>
<223>  Description of Artificial Sequence: Substrate

<220>
<221>  misc_feature
<222>  (12)..(13)
<223>  RNA

<400>  2
aaggtttcct cgucccctggg ca                                                22

<210>  3
<211>  29
<212>  DNA
<213>  Artificial Sequence

<220>
<223>   Description of Artificial Sequence: MNAzyme partzyme A

<400>  3
ccagggaggc tagctggcga cctggaagt                                          29

<210>  4
<211>  28
<212>  DNA
<213>  Artificial Sequence

<220>
<223>   Description of Artificial Sequence: MNAzyme partzyme B

<400>  4
gctcagggaa gacagacaac gaggaaac                                          28


<210>  5
<211>  29
<212>  DNA
<213>  Artificial Sequence

<220>
<223>  Description of Artificial Sequence: Target

<400>  5
acttccaggt cgccctgtct tccctgagc                                         29